# EUROPEAN PATENT SPECIFICATION

(11) **EP 2 878 665 B1**
(45) Date of publication and mention of the grant of the patent: **08.05.2019**
(21) Application number: 13823846.4
(22) Date of filing: 17.05.2013
(51) Int. Cl.: C12N 5/071, A61K 35/12, A61K 35/28, A61L 27/00, C12M 1/00, C12N 5/0775

(54) **TISSUE REGENERATION CONSTRUCT, AND METHOD FOR PRODUCING TISSUE REGENERATION CONSTRUCT**
GEWEBEREGENERATIONSKONSTRUKT UND VERFAHREN ZUR HERSTELLUNG DES GEWEBEREGENERATIONSKONSTRUKTS
CONSTRUCTION POUR RÉGÉNÉRATION DE TISSUS ET SON PROCÉDÉ DE PRODUCTION

(30) Priority: 27.07.2012 JP 2012167382
(43) Date of publication of application: 03.06.2015
(73) Proprietor: GC Corporation, Tokyo 113-0033 (JP)
(72) Inventor: SAKAI, Yuhiro, Tokyo 174-8585 (JP); YAMANAKA, Katsuyuki, Tokyo 174-8585 (JP); YAMAMOTO, Katsushi, Tokyo 174-8585 (JP); SHIGEMITSU, Yusuke, Tokyo 174-8585 (JP); KANEKO, Tadashi, Tokyo 174-8585 (JP)
(74) Representative: Beck Greener LLP
(86) International application number: PCT/JP2013/063827
(87) International publication number: WO 2014/017147

(56) References cited:
- WO-A2-2010/005917
- WO-A2-2012/020412
- JP-A- 2011 160 817
- DATABASE WPI Week 201158 Thomson Scientific, London, GB; AN 2011-K80062 XP002753923, -& JP 2011 160817 A (UNIV TOKYO) 25 August 2011 (2011-08-25)
- KATSUYUKI YAMANAKA ET AL.: 'HAP Gan'yu PLGA Takoshitsu Block o Mochiita Kotsu Soshiki no Saisei' MEDICAL SCIENCE DIGEST vol. 37, no. 8, 30 July 2011, pages 36 - 39, XP008176062

## Description

### BACKGROUND OF THE INVENTION

### Field of the Invention

The present invention relates to a tissue regeneration construct used for effectively regenerating tissue of animals and humans, by means of cells harvested from a living body, and a method for producing the tissue regeneration construct.

Here, the "construct" means a structure constituted by predetermined elements connecting to one another.

### Description of the Related Art

With the progress in cell culturing technique and the progress in medical care, there is an increasing expectation for a method for effectively and promptly curing a target site which is difficult to be cured by conventional medicines or artificial materials, by applying a tissue regeneration treatment by means of various cells including stem cells. The target site is a large-scale tissue defect site which cannot be healed by natural healing power inherent to the living body. For such a site requiring tissue regeneration (target site for tissue regeneration), it is known that a transplant body in which cells and a support (scaffold) capable of holding the cells and having a roll of forming a space for tissue regeneration are combined is applied (Patent Documents 1 to 3).

The application of such a transplant body is recognized as having a certain effect. With this background, researches in this field (preclinical animal experiment and clinical experiment) have been actively carried out. Nowadays, these researches have been progressed as research areas called tissue engineering and regeneration treatment, with increasing kinds of target diseases and cells to be used.

### Citation List

### Patent Literatures

Patent Document 1: Japanese Patent Application Laid-Open No. 2002-209573
Patent Document 2: Japanese Patent Application Laid-Open No. 2005-608
Patent Document 3: WO02/40071

JP 2011 160817 discloses a gel that is suitable for use for regenerating tissue.

WO 2012/020412 discloses a fibrin-based scaffold suitable for supporting a population of cells comprising mesenchymal stem cells (MSC) and/or umbilical tissue derived cells (UTC) at the site of administration for a prolonged period of time.

WO 2010/005917 discloses implants for resurfacing or repairing one or more articular cartilage bearing surfaces of a biological organism include an engineered tissue and a biocompatible porous substrate secured to the engineered tissue for attaching the implant to a native bone of the biological organism.

### SUMMARY OF THE INVENTION

### Problems to be Solved by the Invention

However, with the conventional technique as described in Patent Documents 1 to 3, in some cases, the effect of the treatment is not obtained as expected. Depending on the state of the target site for tissue regeneration, there are some cases in which the transplant body is not engrafted well. As a result, the treatment effect may be limited or the effect cannot be obtained, since the target site for tissue regeneration and the transplant body are not cross linked physically or biologically.

Taking the regeneration of bone tissue as an example, in a case where a transplantation is carried out to a target site for tissue regeneration which has a defect where bone-marrow tissue is exposed, is bleeding, and blood vessels and bone-marrow cells are exposed (that is, the target site has a fresh wound surface), there is a high engraftment capacity from the target site for tissue regeneration to the transplant body. Therefore, it is expected that the transplant body and the target site for tissue regeneration become integrated with each other whereby the tissue regeneration is well carried out. However, the target of which curing is expected by means of the regeneration treatment is not limited to such a site. The target also includes a tissue surface where the tissue is in a state of atrophic after a certain time is passed since the tissue gets damaged, a tissue surface where blood vessels are not exposed, a surface where a part of tissue is necrosed due to a thermal, physical, or chemical damage, a site in a state being difficult to be stabilized because of its physical shape, or a site in which these surfaces and states are mixed. When the site is in such a state, in many cases it is not possible to obtain a sufficient engraftment with the conventional transplant body. Also, even if the surface of the target site for tissue regeneration has a favorable state for tissue regeneration, like the state of the fresh wound surface, in a case where the size of the defect is large, that is, in a case where the area to which regeneration is hoped is large, the tissue regeneration is generally difficult to be done unless a prompt engraftment is carried out.

Accordingly, considering the above problems, an object of the present invention is to provide a tissue regeneration construct having a good engraftment property and with which a stable and favorable regeneration to the target site can be promoted, and a method for producing the tissue regeneration construct.

### Means for Solving the Problems

In order to achieve the above object, the inventors of the present invention have carried out a research and development, with study from various angles. As a result, the inventors have found out that it is effective to make a tissue regeneration construct having a configuration in which an engraftment layer is mediated between the target site for tissue regeneration and the transplant body, in order to make the transplant body engraft to the target site for tissue regeneration. The present invention has been made based on the above finding. Hereinafter the present invention will be described.

A first aspect of the present invention is a tissue regeneration construct which is a member to be applied to a target site for transplantation and regeneration to regenerate tissue, the tissue regeneration construct including: a transplant body; and an engraftment layer arranged overlapping at least a part of an outer surface of the transplant body, wherein the transplant body includes a support and cells for regenerating the tissue, the cells being arranged in at least either one of a space between the supports and a space formed by a pore inside the support, the engraftment layer includes cells for regenerating the tissue and a base material containing fibrin from blood plasma polymerized by coagulation reaction, the base material being for retaining the cells, the base material of the engraftment layer is gelatinous, and the engraftment layer is a layer in which the support does not exist.

Preferably, the cells included in the engraftment layer are in a state of being dispersed with intercellular matrix being decomposed.

Preferably, the base material included in the engraftment layer is at least one selected from the group consisting of gelatin, collagen, extracellular matrix proteins, artificial proteins, and peptide.

Preferably, at least either one of the cells included in the transplant body and the cells included in the engraftment layer are undifferentiated mesenchymal stem cells.

Preferably, at least either one of the cells included in the transplant body and the cells included in the engraftment layer are differentiated cells.

Preferably, at least either one of the cells included in the transplant body and the cells included in the engraftment layer are tissue precursor cells cultured in a state of being differentiated from stem cells.

Preferably, the support of the transplant body is formed containing at least one selected from the group consisting of hydroxyapatite, apatite carbonate, β-TCP, OCP, and calcium phosphate.

Preferably, the support of the transplant body is formed containing at least one selected from the group consisting of PLGA, PLLA, PLC, and artificial polymers having biocompatibility.

Disclosed herein is a method for producing the tissue regeneration construct according to any one of the first to ninth aspects, the method including the steps of: arranging the support in a mold; in the mold in which the support is arranged, pouring a base material suspension containing the cells for regenerating the tissue and the base material for retaining the cells, such that the level of the base material suspension comes above a top surface of the support; and gelling the base material.

Also disclosed herein is a method for producing the tissue regeneration construct, the method including the steps of: arranging the support in a mold such that the support has a gap without having contact with at least one of inner surfaces of the mold; in the mold in which the support is arranged, pouring a base material suspension containing the cells for regenerating the tissue and the base material for retaining the cells; and gelling the base material.

Also disclosed herein is a method for producing the tissue regeneration construct, the method including the steps of: arranging a transplant body in a mold; in the mold in which the transplant body is arranged, pouring a base material suspension containing the cells for regenerating the tissue and the base material for retaining the cells, such that the level of the base material suspension comes above a top surface of the support; and gelling the base material.

Also disclosed herein is a method for producing the tissue regeneration construct, the method including the steps of: arranging the transplant body in the mold such that the transplant body has a gap without having contact with at least one of inner surfaces of the mold; in the mold in which the transplant body is arranged, pouring a base material suspension containing the cells for regenerating the tissue and the base material for retaining the cells; and gelling the base material.

### Effects of the Invention

According to the tissue regeneration construct of the present invention, by transplanting the tissue regeneration construct to the target site for tissue regeneration with the engraftment layer mediating between the target site for tissue regeneration and the transplant body, engraftment is promoted whereby physical and biological cross linkage of the tissue regeneration construct and the target site for tissue regeneration is promptly carried out. With this configuration, stimulation from the tissue to the transplant body such as differentiation, and an early infiltration of vascular tissue are expected, and it becomes easy to supply nutrition and cells from the target site for tissue regeneration, whereby it becomes possible to stably and well regenerate the tissue in a wider range.

Also, according to the method disclosed herein for producing the tissue regeneration construct of the present invention, it is possible to efficiently produce the tissue regeneration construct.

### BRIEF DESCRIPTION OF THE DRAWINGS

Fig. 1 is an appearance diagram of a tissue regeneration construct 10 according to one embodiment;
Fig. 2A is a view showing one situation to explain a production method example 1 of tissue regeneration construct;
Fig. 2B is a view showing another situation to explain the production method example 1 of tissue regeneration construct;
Fig. 3 is a view showing another situation to explain the production method example 1 of tissue regeneration construct;
Fig. 4A is a view showing one situation to explain a production method example 2 of tissue regeneration construct;
Fig. 4B is a view showing another situation to explain the production method example 2 of tissue regeneration construct;
Fig. 5A is a view showing another situation to explain the production method example 2 of tissue regeneration construct;
Fig. 5B is a view showing another situation to explain the production method example 2 of tissue regeneration construct;
Fig. 6A is a view showing another situation to explain the production method example 2 of tissue regeneration construct;
Fig. 6B is a view showing another situation to explain the production method example 2 of tissue regeneration construct after the situation shown in Fig. 6A;
Fig. 7 is an appearance diagram of a tissue regeneration construct 20 according to another embodiment;
Fig. 8A is a view showing one situation to explain a production method example 3 of tissue regeneration construct;
Fig. 8B is a view showing another situation to explain the production method example 3 of tissue regeneration construct;
Fig. 9 is a view showing another situation to explain the production method example 3 of tissue regeneration construct;
Fig. 10 is an appearance diagram of a tissue regeneration construct 30 according to another embodiment;
Fig. 11A is a view showing one situation to explain a production method example 4 of tissue regeneration construct;
Fig. 11B is a view showing another situation to explain the production method example 4 of tissue regeneration construct;
Fig. 12 is a view showing another situation to explain the production method example 4 of tissue regeneration construct;
Fig. 13A is a view to explain the result of Example 1;
Fig. 13B is an enlarged view of a part of Fig. 13A;
Fig. 14A is a view to explain the result of Comparative Example 1;
Fig. 14B is an enlarged view of a part of Fig. 14A.

### DETAILED DESCRIPTION OF THE INVENTION

The functions and benefits of the present invention will be apparent from the following description of modes for carrying out the invention.

### <Tissue regeneration construct 10>

### (Structure of tissue regeneration construct 10)

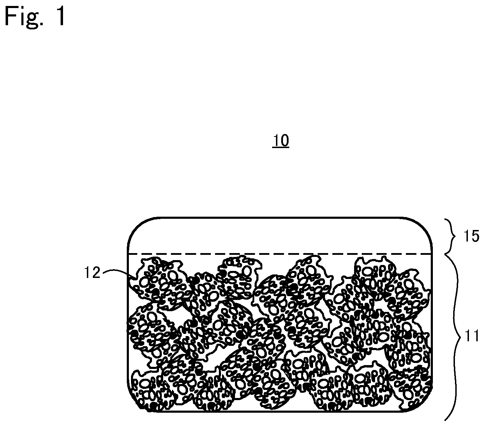

Fig. 1 is a view schematically showing the appearance of the tissue regeneration construct 10 according to one embodiment. As can be seen from Fig. 1, the tissue regeneration construct 10 includes a transplant body 11 and an engraftment layer 15.

The transplant body 11 is configured including a support 12 and cells assuming tissue regeneration.

The support 12 is formed of an artificial or biological polymer or an inorganic material such as calcium phosphate, which has a porous structure with which the cells can be retained thereinside. For example, hydroxyapatite, apatite carbonate, β-TCP, OCP, calcium phosphate, PLGA, PLLA, PLC, and artificial polymers having biocompatibility can be given. The material configuring the support may be nonabsorbable or absorbable. In a case where the material is nonabsorbable, the tissue is regenerated between the support and the pore inside the support. In a case where the material is absorbable, the tissue is also formed in the area created by being absorbed by the support, in addition to the places described above.

Therefore, since the support 12 forms the framework of the transplant body 11, a further efficient tissue regeneration may be carried out if the support 12 is conformed with the shape of the target site for tissue regeneration. From this view point, even though the shape of the support 12 as a whole is not particularly limited, the support 12 may be formed in a shape conformed with the shape of the target site for tissue regeneration where the transplant body 11 is to be transplanted. However, as a versatile configuration, a basic shape such as cubic shape, cuboid shape, hemispherical shape, circular disc shape, and column shape may be prepared. The thickness of the smallest part of the support is preferably 2.2 to 100 mm, more preferably 3 to 100 mm. In the present invention, the support 12 can be largely formed as above; therefore it also adequately meets a case where the target site for tissue regeneration is large.

Also, the support may have a configuration in which powder granulated to have a predetermined particle size is gathered, or may be formed in a block having a predetermined shape. Hereinafter an example in which the support has a configuration in which powder is gathered and an example of the support formed in a block are explained.

In a case where the support 12 has a configuration in which powder is gathered, each powder may be porous or formed in a dense body not including pores. In a case where the powder is porous, the average pore diameter of the pores included in each powder is preferably 50 µm to 500 µm. Regardless of whether the powder is formed in a dense body not including pores or a particle having pores, a space is formed by a gap formed between each powder, which functions as the pore of the porous shape.

Regardless of whether the powder is porous or formed in a dense body, the diameter of the powder is preferably 300 µm to 2000 µm. Also, in a case where the powder is filled in a suitable container, the ratio of the sum of the volume of the gap between the powder and the volume of the pores in the powder, to the volume of the container (porosity when the powder is filled) is preferably 40% to 90%. The porosity when the powder is filled in the container can be calculated by means of a three-dimensional configuration analysis with X-ray CT data.

In a case where the support 12 is formed in a block, the support 12 may be a porous body as an example. It is preferable that the porous body has a pore structure having a pore diameter of 180 µm to 3500 µm, an average pore diameter of 350 µm to 2000 µm, wherein each pore is communicated with one another, and a porosity of 60% to 95%. Also, the support is preferably configured to have 0.05 MPa or more of compressive strength. Here, concerning the pore diameter of the pore of the support, a fine pore having a pore diameter of less than 10 µm where only liquid can go through is not considered, and it means that each pore of 80% or more of the pores each having 10 µm or more of pore diameter in a whole support has a pore diameter of 180 µm to 3500 µm. The "porosity" can be calculated from the weight of the support having the same volume as that of the material lump used for the support, to the weight of the material lump used for the support.

The cells included in the transplant body 11 is not particularly limited as long as the cells are suitable for aimed tissue regeneration being included in the support 12. For example, in a case where regeneration of bone, cartilage, or adipose tissue is carried out: stem cells such as undifferentiated mesenchymal stem cells having a capacity to differentiate to bone, cartilage, or adipose tissue; tissue precursor cells cultured in a state of being differentiated from stem cells such as mesenchymal stem cells; differentiated cells normally configuring the tissue of which the regeneration is intended may be used.

The cells existing inside the support 12 or outer circumferential surfaces of the support 12 construct a regenerated tissue in a space secured by the support 12, by the cells themselves or together with cells of blood vessel and the like entered from outside.

The engraftment layer 15 is a layer arranged overlapping at least a part of an outer surface of the transplant body 11. The engraftment layer 15 does not include the support 12, and includes cells dispersed and retained with a high density to the base material. The engraftment layer 15 is a mixture of the cells and the base material (normally a mixture liquid with a culture solution), and the base material is gelatinous. In applying the tissue regeneration construct to the target site for tissue regeneration, the engraftment layer is arranged having contact with at least a part of a surface of the target site for tissue regeneration where the engraftment is to be promoted to regenerate the tissue.

The kind of the cells included in the engraftment layer 15 is same as or similar to the kind of the cells included in the transplant body 11. However, the cells included in the engraftment layer 15 is dispersed (spread) cells with protein to tie up between the cells (intercellular matrix) being decomposed and loosened up by enzyme treatment and the like. The dispersed cells do not necessarily mean cells in which all cells are dispersed in a uniform state. By including the cells dispersed as above in the engraftment layer 15, it is possible to promote the engraftment of the tissue regeneration construct 10.

This is because the dispersed cells can freely migrate in the base material, which makes it possible for the cells in the engraftment layer 15 to have outgrowth in a direction of the target site for tissue regeneration and a direction of the transplant body 11. Whereby, the transplant body 11 and the target site for tissue regeneration are engrafted, and the transplant 11 and the target site for tissue regeneration are cross linked.

From this viewpoint, it is preferable that a large amount of cells are uniformly dispersed, in order to promote engraftment more efficiently.

The base material included in the engraftment layer 15 has a function of dispersing and retaining the cells to the engraftment layer with a high density and is in a liquid form before becoming gelatinous (hereinafter sometimes simply referred to as "gelated" for short) . As the material of the base material, fibrin (fibrin from blood plasma polymerized by re-coagulation reaction (including PRP: Platelet rich plasma, PPP: Platelet Poor Plasma, and fibrin glue) is used. Each material can be gelated, and the material is gelated when finally formed into the tissue regeneration construct. This gelation makes the base material contract. Not only because the engraftment layer 15 does not include the support , but also because of this contraction, the cell density of the engraftment layer 15 is increased, which makes it possible to sustain a high cell density in the engraftment layer 15, whereby the capacity improvement as the engraftment layer can be expected. Also, this gelation makes a state in which the tissue regeneration construct does not have fluidity when used, whereby it is possible to stably apply the tissue regeneration construct to the target site for tissue regeneration.

The thickness of the engraftment layer 15 is larger than 0 mm, preferably 1 mm or less, more preferably 0.5 mm or less, further preferably 0.2 mm or less. Alternatively, to the size T of the transplant body 11 in the same direction as the direction of the thickness t of the engraftment layer 15, preferably t/T is 0.03 to 1.0. More preferably t/T is 0.03 to 0.5.

The cell density of the engraftment layer 15 is better as the cell density per dimension having contact with the target site for tissue regeneration is more concentrated, and preferably 0.2×10⁴ cell/mm² or more, more preferably 0.5×10⁴ cell/mm² or more, further preferably 1.0×10⁴ cell/mm² or more, and most preferably 1.5×10⁴ cell/mm² or more.

The support 12 does not exist in the engraftment layer 15, and the cells exist in the engraftment layer 15 with a high density. Therefore, the local concentration of liquid factor (proteins such as growth factor) produced by the cells is also high, whereby it is possible to promote cell mobilization from the target site for tissue regeneration and to add an activating stimulus to the cells.

In the tissue regeneration construct 10 described above, the transplant body 11 and the engraftment layer 15 may be integrally produced to form the tissue regeneration construct 10. The tissue regeneration construct 10 may also be formed by: arranging only the engraftment layer 15 on a surface of a site (for example, may be the target site for tissue regeneration) in advance; then directly contacting the transplant 11 thereto. Hereinafter, examples of the production method of the tissue regeneration construct 10 will be described.

(Production method example 1 of tissue regeneration construct)

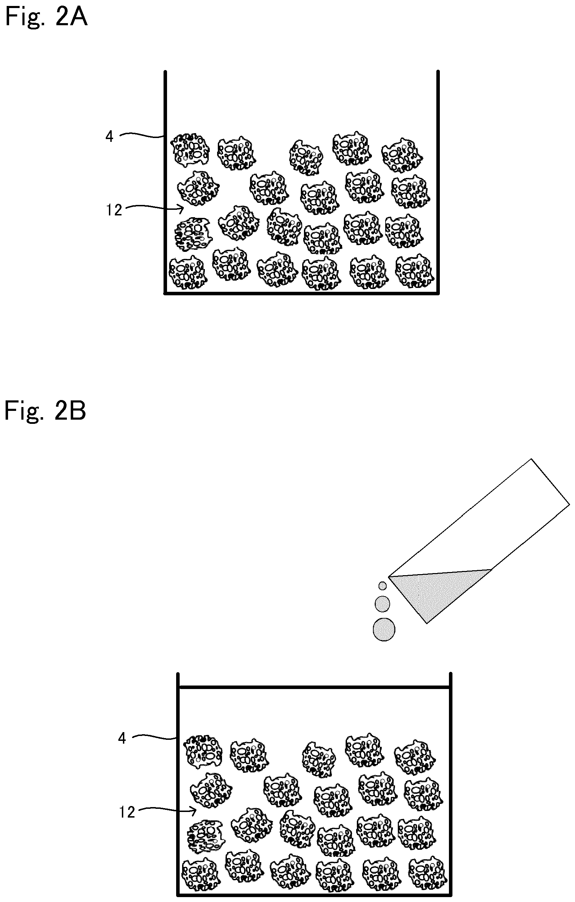

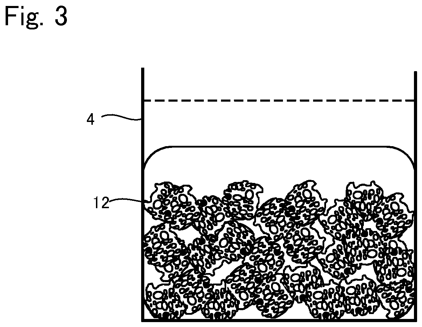

Figs. 2 and 3 are schematic views to explain the production method example 1 of the tissue regeneration construct 10.

As shown in Fig. 2A, the support 12 in a powder form is put in a mold 4 so that the support 12 has a predetermined volume.

Next, a suspension (base material suspension) in which the cells for tissue regeneration are dispersed in the base material is poured in the mold 4. At this time, as shown in Fig. 2B, the suspension is poured such that the top surface of the suspension comes above the top surface of the support 12 arranged in the mold 4. Whereby, the bottom part in the mold 4 becomes a layer of the support 12 and the suspension, and the top part becomes a layer of only the suspension not including the support 12.

Next, the content of the mold 4 is well pipetted up and down so that the suspension reaches between the support 12 and the pores inside the support 12.

Next, the gelation of the base material is carried out. The fibrin from blood plasma is used for the material of the base material and calcium chloride aqueous solution, or thrombin aqueous solution in some cases is added to the base material and pipetted up and down. Whereby, the base material is formed into a gel.

By carrying out the gelation, the contraction is occurred as shown in Fig. 3, and the cell density in the engraftment layer is increased. Also, the cells are retained because the cells are closed in three-dimensional mesh because of the gelation. The dashed line shown in Fig. 3 is the level of the base material suspension before gelation. As described above, it is shown that the contraction is occurred by the gelation.

Whereby, in the mold 4, the tissue regeneration construct 10 in which: the top part is the engraftment layer 15 consisting of the base material in a gel form and the cells; and the bottom part is the transplant body 11 consisting of the support 12, the cells, and the base material is formed. In this example, the same cells and the same base material are used for the transplant body 11 and the engraftment layer 15.

### (Production method example 2 of tissue regeneration construct)

Next, another example of the production method of the tissue regeneration construct 10 (production method example 2 of tissue regeneration construct) will be described.

This is a method of producing the tissue regeneration construct 10 by: arranging only the engraftment layer 15 on the surface of the target site for tissue regeneration in advance; then directly contacting the transplant body 11 thereto.

In this method, a material prepared in advance by: uniformly dispersing and mixing the support which is an aggregation of powder and the base material suspension of the cells; then molding the obtained mixture integrally by means of galation and the like, or a material prepared in advance by introducing the cells to the inner pores of the support formed in a porous block, is used as the transplant body 11. The transplant body 11 may be cultured with the cells introduced therein.

Here, the "material prepared by: uniformly dispersing and mixing the support which is an aggregation of powder and the base material suspension of the cells; then molding the obtained mixture integrally by means of galation and the like" may be formed by, as explained in the production method example 1 of tissue regeneration construct: pouring the suspension in the support which is an aggregation of powder filled in the mold; then gelling the resultant material in the same manner as in the production method example 1 of tissue regeneration construct. In this regard, if the top surface of the support which is an aggregation of powder and the top surface of the poured suspension are made to be positioned substantially same, it is possible to produce only the transplant body.

Also, the tissue regeneration construct 10 formed in the production method example 1 of tissue regeneration construct may be used as it is.

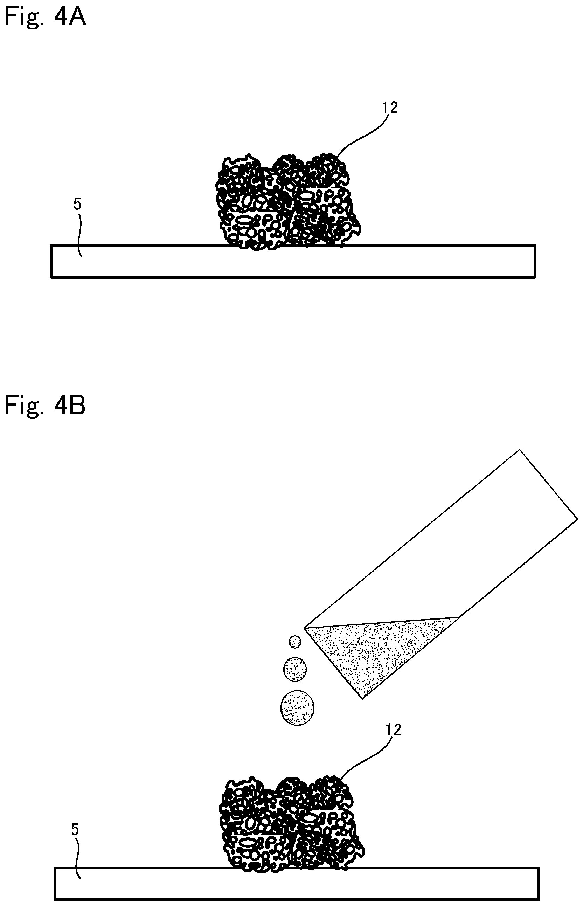

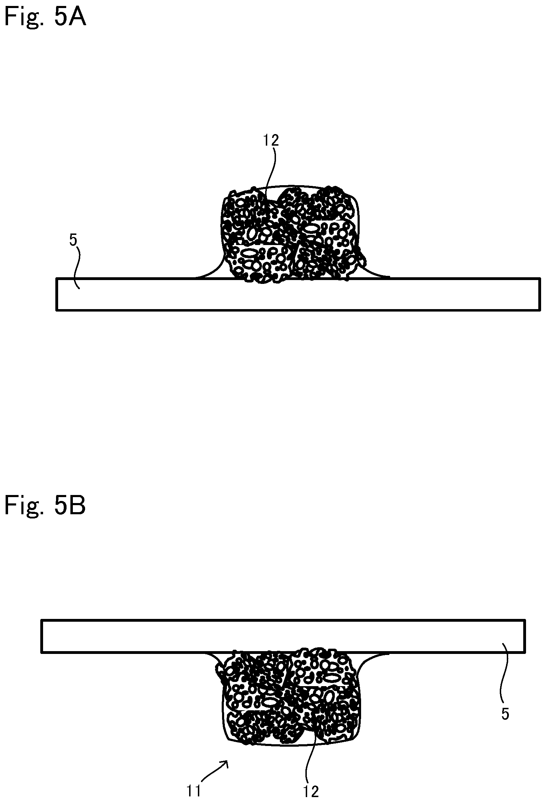

Regarding the "material prepared in advance by introducing the cells to the inner pores of the support formed in a porous block", the cells are preferably uniformly dispersed in the support. To this end, press fitting, defoaming, and other methods may be employed. In specific, the material can be made as follows for example. Figs. 4 and 5 show schematic views to explain the production process.

First, as shown in Fig. 4A, the support 12 is placed on a holding plate 5 which is a resin plate or a glass plate, each having a contact angle to water of 15 to 90°.

Next, as shown in Fig. 4B, the base material suspension is introduced to the support 12 by dropping or injection for example. Whereby, the suspension permeates into the support 12 and fills up the whole body in the support 12, as shown in Fig. 5A.

Further, the holding plate 5 and the support 12 including the suspension are reversed from the state shown in Fig. 5A to have the state shown in Fig. 5B, such that the holding plate 5 comes on the top and the support 12 comes at the bottom. That is, when seen in the gravity direction, the holding plate 5 is on the upper side of the support 12, and they are kept still in the air in a state that the weight of the holding plate 5 is not applied to the support 12. With the holding plate 5 and the support 12 kept still in this state, the cells that have been introduced are adhered to the inner walls of the pores in the support 12, thereby completing seeding thereof. The obtained is the transplant body 11. The time to keep the support 12 still in the air in order to adhere the cells thereto varies depending on the material of the support 12 and the kind of cell to be seeded; however, it is generally 20 to 300 minutes.

On the other hand, aside from the transplant body 11 described above, a member to be the engraftment layer 15 is produced. That is: the base material suspension in which the cells to be used for the engraftment layer 15 are dispersed to the base material is prepared and poured in a mold so as to have a required dimension and thickness; thereafter, the base material is gelated. The gelation may be carried out by the same method as the production method example 1 of tissue regeneration construct. Whereby, the member to be the engraftment layer 15 is prepared separately from the transplant body 11.

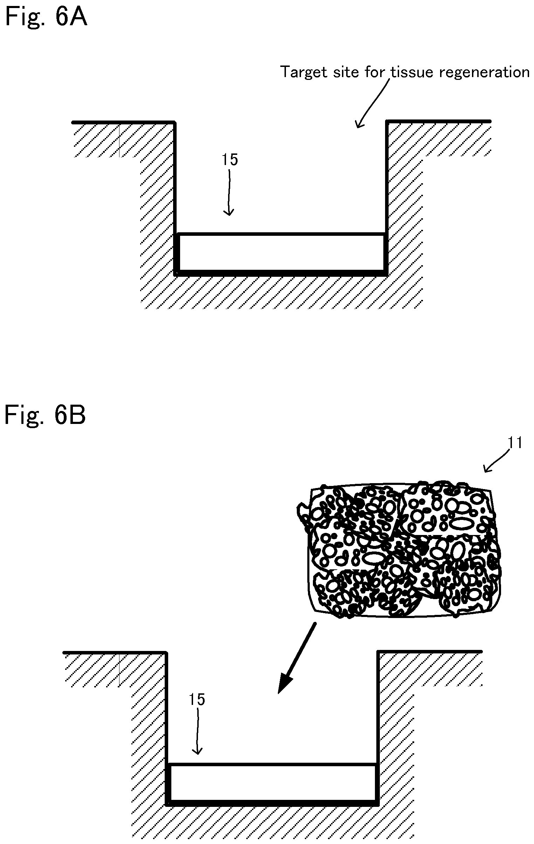

As described above, the transplant body 11 and the engraftment layer 15 are separately prepared, then the target site for tissue regeneration is exposed. The tissue regeneration construct 10 is formed thereto. Fig. 6 shows views for explanation. As shown in Fig. 6A, the engraftment layer 15 produced as above is firstly arranged to at least a part of the surface of the exposed target site for tissue regeneration, where engraftment is to be promoted to regenerate tissue. Next, as shown in Fig. 6B, the transplant body 11 is put on the arranged engraftment layer 15, whereby the production of the tissue regeneration construct 10 is completed on the target site for tissue regeneration. In the tissue regeneration construct 10 produced in this example, the kind of the cells of the transplant body 11 and the kind of the cells of the engraftment layer 15 may be same or different. Also, in the tissue regeneration construct 10 produced in this example, the kind of the base material included in the transplant body 11 and the kind of the base material included in the engraftment layer 15 may be same or different.

Also, an example in which the tissue regeneration construct is formed directly to the target site for tissue regeneration is explained here; however, the tissue regeneration construct does not necessarily need to be directly formed to the target site for tissue regeneration. For example, the tissue regeneration construct may be similarly produced in a mold or on a plate.

### <Tissue regeneration construct 20>

### (Structure of tissue regeneration construct 20)

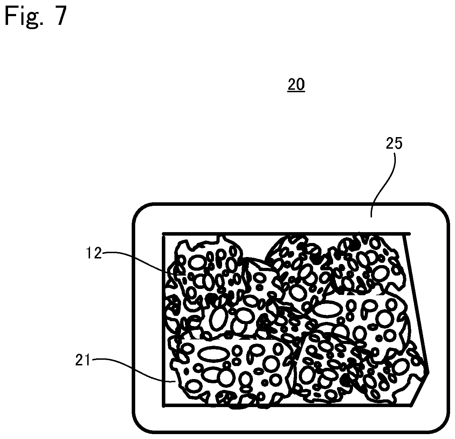

Fig. 7 schematically shows an appearance of a tissue regeneration construct 20 according to another embodiment. As can be seen from Fig. 7, the tissue regeneration construct 20 also includes a transplant body 21 and an engraftment layer 25. In the tissue regeneration construct 20, the engraftment layer 25 is provided to a plurality of outer surfaces of the transplant body 21. The outer surface of the transplant body 21 to which the engraftment layer 25 is provided is not particularly limited; the engraftment layer 25 may be provided to all surfaces of the transplant body 21, or to a plurality of surfaces in a part of the transplant body 21.

The materials to configure the transplant body 21 and the engraftment layer 25 are same as that of the transplant body 11 and the engraftment layer 15 described above.

The method for producing the tissue regeneration construct 20 is not particularly limited, and for example it may be produced as follows.

### (Production method example 3 of tissue regeneration construct)

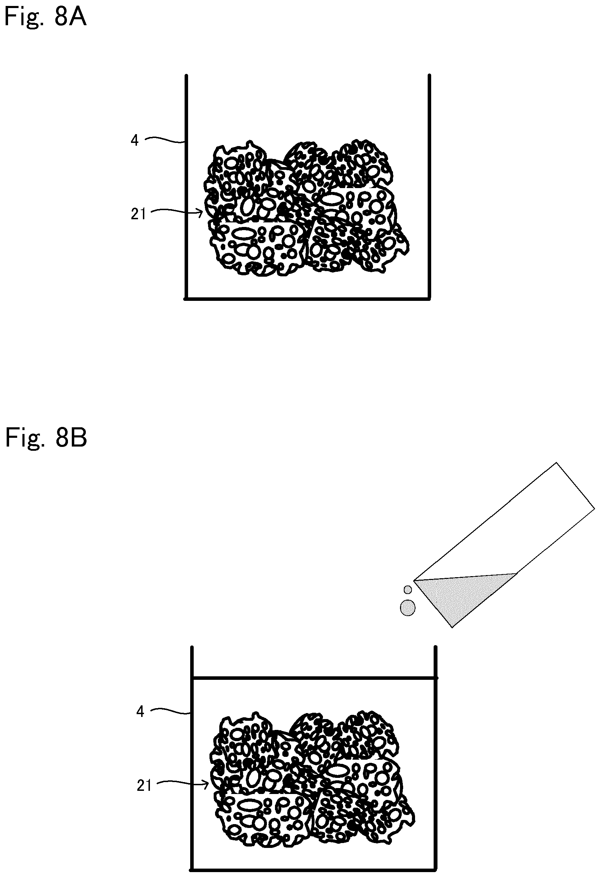

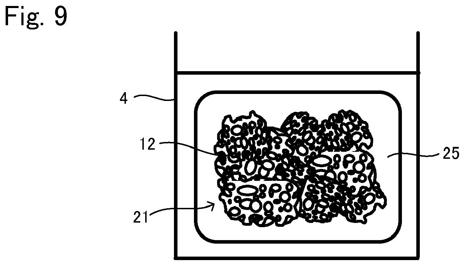

Figs. 8 and 9 show views to explain the production method example 3 of tissue regeneration construct.

In this production method example 3, a material prepared in advance by: uniformly dispersing and mixing the support which is an aggregation of powder and the base material suspension of the cells; then molding the obtained mixture integrally by means of galation and the like, or a material prepared in advance by introducing the cells to the inner pores of the support formed in a porous block, is used as the transplant body 21. The transplant body 21 may be cultured with the cells introduced thereto. The production of the transplant body 21 may be carried out in the same manner as in the production method example of the transplant body 11 explained in the production method example 2 of tissue regeneration construct described above. Alternatively, the tissue regeneration construct 10 produced in the production method example 1 of tissue regeneration construct may be used as it is.

The produced transplant body 21 is put in a mold 4 having a depth larger than the height of the transplant body 21, as schematically shown in Fig. 8A. In this regard, the transplant body 21 is arranged such that: regarding a surface of the transplant body 21 where the engraftment layer 25 is to be formed, a gap is provided between the surface and a wall surface of the mold 4; on the other hand, regarding a surface of the transplant body 21 where the engraftment layer 25 is not to be formed, the surface is in contact with an inner surface of the mold 4. In a case where the engraftment layer 25 is needed to an outer surface of the transplant body 21 which faces the bottom surface of the mold 4, a supporter which supports the transplant body 21 from the bottom surface of the mold 4 by a point or a line can be used. Alternatively, the transplant body 21 may be held from the above by means of tweezers or the like to maintain a state in which the transplant body 21 is still in the air from the bottom surface of the mold 4.

Thereafter, the base material suspension in which the cells to be used for the engraftment layer 25 are dispersed is poured in the mold 4, as shown in Fig. 8B. The engraftment layer 25 is formed to the height where is immersed in the suspension. Therefore, by setting the amount of the suspension such that the level of the suspension comes higher than the height of the transplant body 21, the engraftment layer 25 can also be formed on a top surface side of the transplant body 21. As described above, the engraftment layer 25 is formed as appropriate.

After that, the gelation of the base material is carried out. For the gelation, the same method as the method explained in the production method example 1 of tissue regeneration construct described above can be applied.

With this gelation, contraction described above is occurred as shown in Fig. 9, and the cell density in the engraftment layer is increased. Also, since the cells are closed in three-dimensional mesh because of the gelation, the cells are retained therein. The dashed line shown in Fig. 9 is the level of the base material suspension before gelation. As can be seen, contraction is occurred by the gelation.

As described above, the tissue regeneration construct 20 in which the engraftment layer 25 is formed on arbitrary surfaces surrounding the transplant body 21 can be efficiently produced. In the tissue regeneration construct 20 produced in this example, the kind of the cells included in the transplant body 21 and the kind of the cells included in the engraftment layer 25 may be same or different. Also, in the tissue regeneration construct 20 produced in the example, the kind of the base material included in the transplant body 21 and the kind of the base material included in the engraftment layer 25 may also be same or different.

### <Tissue regeneration construct 30>

### (Structure of tissue regeneration construct 30)

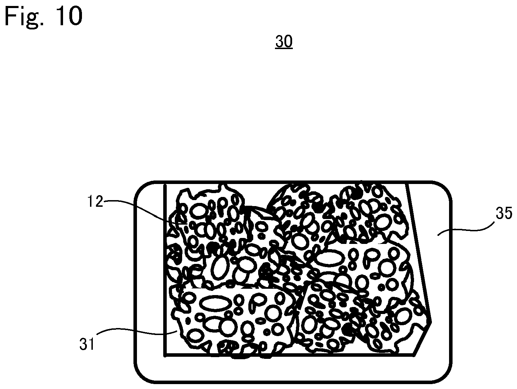

Fig. 10 schematically shows an appearance of a tissue regeneration construct 30 according to another embodiment. As can be seen from Fig. 10, the tissue regeneration construct 30 also includes a transplant body 31 and an engraftment layer 35. In the tissue regeneration construct 30, the engraftment layer 35 is provided to a plurality of outer surfaces of the transplant body 31, and is not provided to at least one surface. The outer surface of the transplant body 31 where the engraftment layer 35 is provided is not particularly limited, and may be whole surface except one surface, or the transplant body 31 may have a plurality of outer surfaces where the engraftment layer 35 is not provided.

The materials to configure the transplant body 31 and the engraftment layer 35 are same as that of the transplant body 11 and the engraftment layer 15 described above.

The method for producing the tissue regeneration construct 30 is not particularly limited, and it may be produced as follows for example.

### (Production method example 4 of tissue regeneration construct)

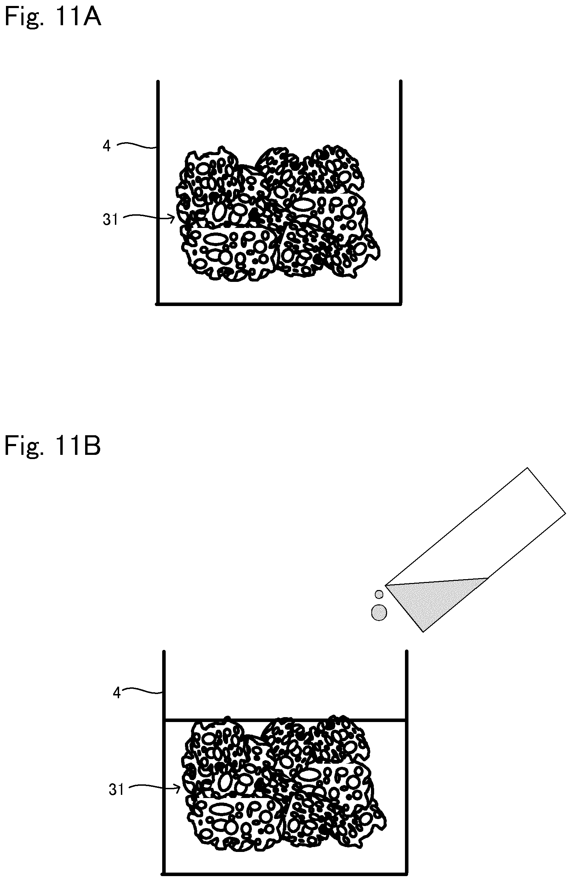

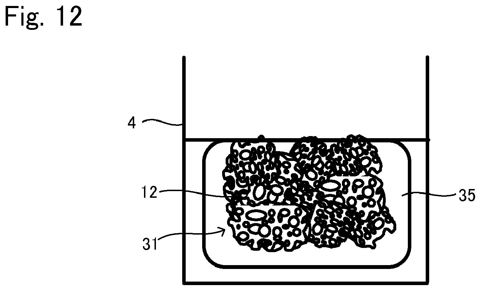

Figs. 11 and 12 show views to explain production method example 4 of tissue regeneration construct.

In the production method example 4, a material prepared in advance by: uniformly dispersing and mixing the support which is an aggregation of powder and the base material suspension of the cells; then molding the obtained mixture integrally by means of galation and the like, or a material prepared in advance by introducing the cells to the inner pores of the support formed in a porous block, is used as the transplant body 31. The transplant body 31 may be cultured with the cells introduced thereto. The production of the transplant body 31 may be carried out in the same manner as in the production method example of the transplant body 11 explained in the production method example 2 of tissue regeneration construct. Alternatively, the tissue regeneration construct 10 produced in the production method example 1 of tissue regeneration construct may be used as it is.

The produced transplant body 31 is put in the mold 4 as schematically shown in Fig. 11A. In this regard, the transplant body 31 is arranged such that: regarding a surface of the transplant body 31 where the engraftment layer 35 is to be formed, a gap is provided between the surface and an inner surface of the mold 4; on the other hand, regarding a surface of the transplant body 31 where the engraftment layer 35 is not to be formed, the surface is in contact with an inner surface of the mold 4. In a case where the engraftment layer 35 is needed on the outer surface of the transplant body 31 which faces the bottom surface of the mold 4, a supporter which supports the transplant body 31 from the bottom surface of the mold 4 by a point or a line can be used. Alternatively, the transplant body 31 may be held from the above by means of tweezers or the like to maintain a state in which the transplant body 31 is still in the air from the bottom surface of the mold 4.

Thereafter, the base material suspension in which the cells to be used for the engraftment layer 35 are dispersed is poured in the mold 4, as schematically shown in Fig. 11B. In this example, since the engraftment layer 35 is formed to the height where is immersed in the suspension, the level of the suspension is made to be same as the top surface of the transplant body 31. With this configuration, the engraftment layer 35 is not formed on a top surface side of the transplant body 31 at least in Fig. 11b.

After that, the gelation of the base material is carried out. The same method as in the method explained in the production method example 1 of tissue regeneration construct described above can be applied to the gelation.

This gelation causes the above contraction as shown in Fig. 12, whereby the cell density of the engraftment layer is increased. Also, the cells are closed in the three-dimensional mesh by the gelation, whereby the cells are retained therein. The dashed line shown in Fig. 12 is the level of the suspension before gelation. As can be seen, the contraction is occurred by the gelation.

As described above, the tissue regeneration construct 30 in which the engraftment layer 35 is formed on the predetermined surfaces surrounding the transplant body 31 may be efficiently produced. In the tissue regeneration construct 30 produced in this example, the kind of the cells included in the transplant body 31 and the kind of the cells included in the engraftment layer 35 may be same or different. Also, in the tissue regeneration construct 30 produced in this example, the kind of the base material included in the transplant body 31 and the kind of the base material included in the engraftment layer 35 may be same or different.

### <Function of tissue regeneration construct>

According to the tissue regeneration constructs 10, 20, and 30, in addition to the transplant body provided with the support retaining the cells that are the object to be regenerated, the engraftment layer including the cells with a high density but not including the support is provided. By arranging the engraftment layer having contact with at least a part of the surface of the target site for tissue regeneration, where the engraftment is promoted to regenerate the tissue, the engraftment is promoted and a physical and biological cross linkage of the tissue regeneration construct and the target site for tissue regeneration is promptly carried out, whereby it is possible to promote the regeneration.

With this configuration, it is expected that the transplant body is activated from tissue with a stimulation such as differentiation, and that an early infiltration of vascular tissue and supply of nutrition and cells from the target site for tissue regeneration will occur in easier. It also can be considered that it is possible to promote cell mobilization from the target site for tissue regeneration by the liquid factor (proteins such as growth factor) produced by the cells and to add an activating stimulus to the cells.

Also, since the cells in the engraftment layer are dispersed by enzyme treatment and the like, the cells can freely migrate in the engraftment layer and can have outgrowth toward the target site for tissue regeneration and the transplant body, whereby the engraftment and cross linkage of the transplant body and the target site for tissue regeneration are promoted.

Also, in a case where the target site for tissue regeneration is relatively large, migration and outgrowth of the cells in the engraftment layer and the cells in the target site for tissue regeneration are efficiently occurred via the engraftment layer, which stimulates well the cells of the transplant body and its effect is efficiently transmitted to the cells in the whole area of the transplant body, whereby the regeneration is promoted. Therefore, according to the tissue regeneration constructs 10, 20, and 30, it is possible to widen the tolerance of the size of the target site for tissue regeneration.

### Examples

Hereinafter the present invention will be further specifically explained based on Examples; however the present invention is not limited to Examples.

### <Example 1>

In Example 1, a tissue regeneration construct was produced by means of the following process, and thereafter transplanted.

### (Culture of mesenchymal stem cell derived from bone marrow of rat)

Femurs and tibias of an F344 rat of 4 weeks old were collected. Bone marrow cells (from 2 femurs and 2 tibias) obtained by flushing out the bone marrow with a culture solution were seeded on a culture medium supplemented with 30 ml of αMEM medium including 10% FBS and 1% penicillin streptomycin. The cells were cultured to be proliferated under the presence of 5% carbon dioxide gas at 37°C. The culture medium was changed on the third day, to remove non-adherent cells. After that, the culture medium was changed every third days. From the time when the culture medium was changed for the first time, 3 ng/ml of bFGF was added to the culture medium. Around the 10th day, it was confirmed that the cells were proliferated to be nearly confluent. Thereafter, the culture medium was removed, and the cells were incubated for 2 minutes with trypsin (0.05%) and EDTA (0.2 mM) and subjected to vibration, to be removed from the culture medium and isolated as soon as possible. Immediately after that, a culture medium was given to the cells to stop the activity of trypsin.

The number of cells was measured and the cells were subcultured at a density of 5000 cells/cm². The cells were further cultured for 5 days, thereafter removed from the culture medium as described above and dispersed (isolated) to be applied to the following experiment.

### (Production of tissue regeneration construct)

Powder of hydroxyapatite porous body (ϕ0.5 mm to ϕ2.0 mm) to be used as the support was filled in a LAB-TEK CHAMBER SLIDE 16 well (used as a mold: inner diameter of 7 mm, bottom area of 38.5 mm²) manufactured by Nunk, such that the height of the powder was 3 mm per well. The inner diameter of the well was 7 mm and the volume was 115 µl. The porosity of the support in a state of being filled in the well was 75%, which meant that 86.25 µl of empty space was existed in the entire volume of 115 µl.

To the well, a cell suspension in which 200×10⁴ cells of the mesenchymal stem cells derived from the bone marrow of the rat produced as above was suspended in 120 µl of blood plasma derived from the F344 rat was added and pipetted up and down, so that the cells reaches the entire area. Thereto, 12 µl of 3.3% aqueous solution of calcium chloride was added and pipetted up and down so that the aqueous solution spreads well. The obtained was incubated at 37°C, to promote polymerization of fibrinogen in the blood plasma, which is a coagulation reaction, to make fibrin. As a result, a lump in which the cells and the support body were included in the blood plasma was formed in the well. On the top surface of the support filled in the well, a layer having a thickness of approximately 1.2 mm in which the cells are included in the coagulated fibrin without having the support was overlapped. This layer was the engraftment layer. The number of the cells in the engraftment layer was approximately 69×10⁴ cells and approximately 1.8×10⁴ cells/mm² per square measure.

### (Transplantation)

Hair at the skull part of the rat was cut under general anesthesia with isoflurane, and disinfection was carried out by Isodine. Thereafter a scalpel was put into the skull part to a depth around 1.5 cm so that the scalpel reaches periosteum. A periosteal elevator was put into the incision line of the skull part to separate the periosteum and epithelium together in a tunnel shape from the bone. The produced tissue regeneration construct was maintained at 37°C until just before transplantation. The walls of the well of the chamber slide were removed and the tissue regeneration construct was taken out just before transplantation. Thereafter, the engraftment layer at the top portion of the tissue regeneration construct was arranged in the separated tunnel, toward the skull bone. In this Example 1, it was expected that the tissue regeneration construct was engrafted to the skull bone and the area where the tissue regeneration construct was transplanted became bone, whereby the bone outgrowth was found such that the bone had a shape of being risen up.

After the transplantation, the incision line was sutured and the rat was put back in a cage.

### <Comparative Example 1>

A tissue regeneration construct produced in the same manner as in Example 1 described above was prepared. The tissue regeneration construct was arranged and engrafted on the skull bone of a rat as in the same manner. However, in Comparative Example 1, the engraftment layer was not arranged toward a skull bone side but arranged toward a periosteum side positioned upside.

That is, in Comparative Example 1, the number of given cells were same as in the Example 1 described above, whereas the engraftment layer did not have contact with the surface of the target site for tissue regeneration, where the engraftment was to be promoted to regenerate the tissue, thereby not having mediacy of the engraftment layer. Therefore, Comparative Example 1 was an example of a tissue regeneration construct in which the engraftment layer was not substantially provided. After the transplantation, the incision line was sutured in the same manner as above and the rat was put back in a cage.

### [Histological evaluation]

### (Preparation of sample)

After an eight-week healing period, the experimental animals in Example 1 and Comparative Example 1 were slaughtered and tissues were collected therefrom. The tissues were applied to formalin fixation and embedded in paraffin. Thereafter each of the center portions of the transplanted tissue regeneration constructs was thinly cut to produce 5 samples. After that, HE stain was applied to the samples and a histological evaluation was carried out thereto.

### (Results)

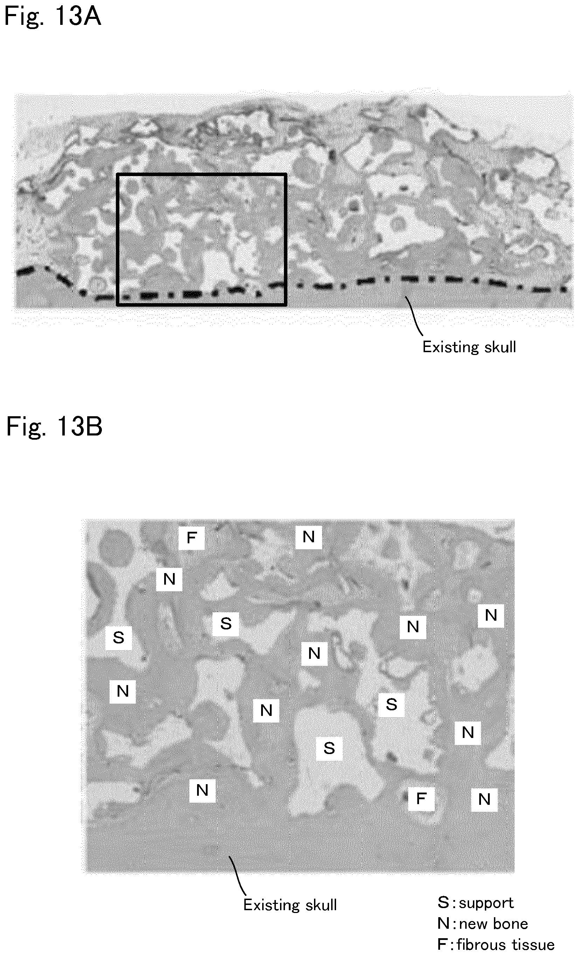

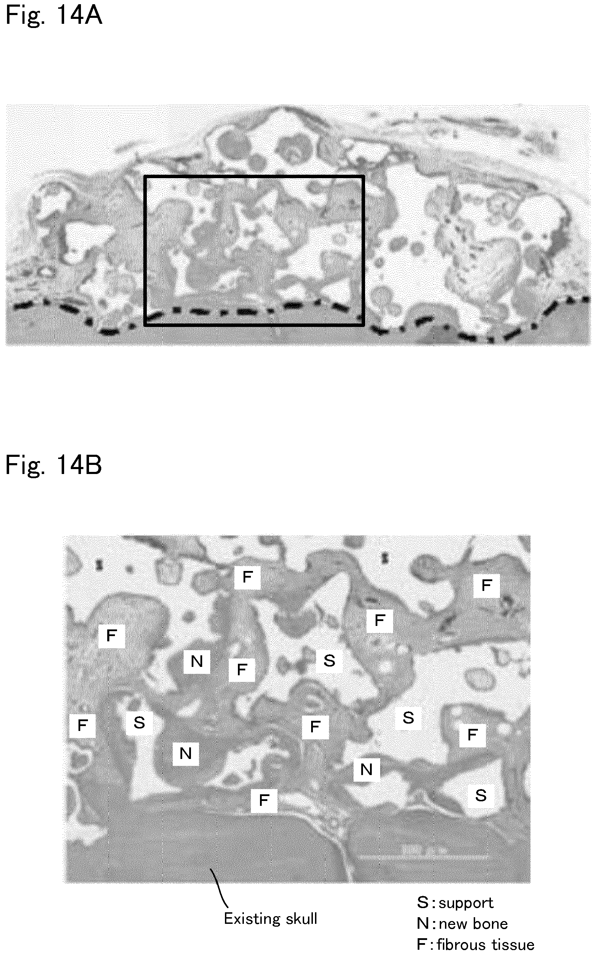

Fig. 13 shows one of the samples of Example 1. Fig. 13A is an entire view and Fig. 13B is an enlarged view of the portion enclosed by a box shown in Fig. 13A. Fig. 14 shows one of the samples of Comparative Example 1 in the same manner as in Fig. 13.

As a result, in Example 1, a bone formation in which the skull bone and the tissue regeneration construct continue in a wide range was confirmed. It can be seen that a lot of new bones (shown by "N") were formed in Example 1, as can be seen from Fig. 13B. Also, highly activated osteoblast cells were arranged on the surface of the new bone area, whereby further new bone formation was expected.

On the other hand, in Comparative Example 1, bone formation was only sporadically confirmed at the circumference of the support in the construct. As can be seen from Fig. 14B, the area where the bone formation was not confirmed was filled by fibrous tissue (shown by "F").

Further, the following results were obtained quantitatively:
(1) in the observation of the above thin slice samples, in each 5 samples, 5 samples (all) of Example 1 and only 1 sample of Comparative Example 1 had 60% or more of area ratio of new bone in the area excluding the support.
(2) in the observation of the above thin slice samples, in each 5 samples, 5 samples (all) of Example 1 and only 1 sample of Comparative Example 1 had 50% or more of the length of the interface where the cross linkage of bone from the existing skull to the support in the tissue regeneration construct was shown, with the bottom surface of the tissue regeneration construct and the existing skull continuously grafted by new bones.

From the above results, it was confirmed that the bone formation was promoted by the tissue regeneration construct provided with the engraftment layer, the engraftment layer mediating between the tissue regeneration construct and the target site for tissue regeneration. Whereby it became clear that the tissue regeneration construct according to the present invention is effective for outgrowth of bone.

### <Example 2>

In Example 2, a tissue regeneration construct was produced by means of the following process and transplanted.

### (Culture of mesenchymal stem cell derived from bone marrow of rat)

Femurs and tibias of an F344 rat of 4 weeks old were collected. Bone marrow cells (from 2 femurs and 2 tibias) obtained by flushing out the bone marrow with a culture solution were seeded on a culture medium supplemented with 30 ml of αMEM medium including 10% FBS and 1% penicillin streptomycin. The cells were cultured to be proliferated under the presence of 5% carbon dioxide gas at 37°C. The culture medium was changed on the third day, to remove non-adherent cells. After that, the culture medium was changed every third days. From the time when the culture medium was changed for the first time, 3 ng/ml of bFGF was added to the culture medium. Around the 10th day, it was confirmed that the cells were proliferated to be nearly confluent. Thereafter, the culture medium was removed, and the cells were incubated for 2 minutes with trypsin (0.05%) and EDTA (0.2 mM) and subjected to vibration, to be removed from the culture medium and isolated as soon as possible. Immediately after that, a culture medium was given to the cells to stop the activity of trypsin.

The number of cells was measured and the cells were subcultured at a density of 5000 cells/cm². The cells were further cultured for 5 days, thereafter removed from the culture medium as described above and dispersed (isolated) to be applied to the following experiment.

### (Production of tissue regeneration construct)

A 1 ml syringe (inner diameter: 4.5 mm) manufactured by NIPRO with its tip being cut was prepared as a mold. A tip of a piston was applied to the portion having 10 mm of distance from the end of the syringe where the tip was cut, and powder of hydroxyapatite porous body (ϕ0.5 mm to ϕ2.0 mm) to be used as the support was filled to the syringe. The volume of the syringe from the end where the tip was cut to the tip of the piston was 159 µl. Since the porosity of the support when filled in container was 75%, 119 µl was a gap space. To the gap space, a cell suspension in which 200×10⁴ of the mesenchymal stem cell derived from the bone marrow of the F344 rat cultured as above was suspended to 107 µl of blood plasma of the F344 rat was added. The obtained was pipetted up and down with a syringe with a 27G needle so that the cells evenly spread throughout. Further, 12 µl of 3.3% calcium chloride aqueous solution was added thereto and pipetted up and down so as to spread well. The obtained was incubated at 37°C to promote the polymerization of the fibrinogen in the blood plasma which is a coagulation reaction. As a result, a lump (main body of the transplant body) in which the cells and the support were contained in the fibrin from blood plasma was formed in the 1 ml syringe. In this case, there was nothing corresponding to the engraftment layer existed at this point.

Next, the piston of the syringe was pushed and the transplant body produced as above was taken out as a lump having a cylindrical shape. Then, the lump was put in a sterilized ϕ 10 mm cylindrical container, held by tweezers and kept in a state being still in the air having 3 mm of distance from the bottom surface of the container in a circular shape. Thereto, a cell suspension in an amount of 1097 µl, in which 200×10⁴ cells/1000 µl of the above mesenchymal stem cell derived from the bone marrow of the F344 rat was suspended to the blood plasma of the F344 rat was poured and well pipetted up and down. Further, 110 µm of 3.3% calcium aqueous solution was added thereto and well pipetted up and down. At a right time at which the coagulation reaction was progressed, the obtained was further incubated at 37°C to promote the polymerization of the fibrinogen in the blood plasma, which is a coagulation reaction. Whereby, the engraftment layer having a thickness of 3 mm was formed on the top and at the bottom of the transplant body. Also, at the same time the engraftment layer was formed in the side face of the portion of the transplant body in a cylindrical shape. The engraftment layer contracted as time passed, resulted in having a thickness of approximately 0.3 mm or less. The cell density of the engraftment layer made as above existing on the top and the bottom of the tissue regeneration construct having a cylindrical shape was approximately 0.5×10⁴ cells/mm².

### (Transplantation and evaluation thereof)

The epithelium of femur of the F344 rat was incised, and fascias and muscles surrounding the femur were split, whereby the femur was exposed. The exposed femur was fixated by an external fixator, whereby a bone defect having a gap of 10 mm was produced. To this model of bone defect, the above cylindrical tissue regeneration construct was inserted to be transplanted such that the engraftment layer on the top and the bottom of the cylindrical shape was in contact with cross sectional areas of the bones.

The tissue regeneration construct was one lump, and firmly fixed by being sandwiched by the cross sectional areas of the bones .

The muscles and the fascias were sutured, and the epithelium was also sutured to thereby seal the wound. The healing process was evaluated by means of X-raying of the defect part at the first, second, fourth, and eighth week. At the fourth week after transplantation, the boundary between the end of the cross sections of the bones and the end of the tissue regeneration construct became unclear, which suggested that cross linkage of bone was occurred. Also, the femur was extracted at the eighth week after transplantation and applied to µCT and histopathologic evaluation.

As a result, it was confirmed that the tissue regeneration construct and the remaining femur were cross liked. Also, a matured bone formation was found to the transplant body of the tissue regeneration construct in a manner to cover the hydroxyapatite powder used as the support, and a formation of myeloid-like tissue was partly found. It seemed that the whole tissue regeneration construct transplanted replaced the femur as one lump.

From the above, it was confirmed that it is possible to cure a defect of rat femur which is such a large-scale defect that it is impossible to be naturally cured, by transplanting the tissue regeneration construct of the present invention.

### Description of the Reference Numerals

- 10, 20, 30: tissue regeneration construct
- 11, 21, 31: transplant body
- 12: support
- 15, 25, 31: engraftment layer

## Claims

1. A tissue regeneration construct (10, 20, 30) which is a member to be applied to a target site for transplantation and regeneration to regenerate tissue, the tissue regeneration construct (10, 20, 30) comprising:
a transplant body (11, 21, 31); and
an engraftment layer (15, 25, 35) arranged overlapping at least a part of an outer surface of the transplant body,
wherein
the transplant body (11, 21, 31) comprises a support (12) and cells for regenerating the tissue, the cells being arranged in at least either one of a space between the supports and a space formed by a pore inside the support,
the engraftment layer (15, 25, 35) comprises cells for regenerating the tissue and a base material containing fibrin from blood plasma polymerized by coagulation reaction, the base material being for retaining the cells,
the base material of the engraftment layer is gelatinous, and
the engraftment layer (15, 25, 35) is a layer in which the support (12) does not exist.

2. The tissue regeneration construct (10, 20, 30) according to claim 1, wherein the cells comprised in the engraftment layer (15, 25, 35) are in a state of being dispersed with intercellular matrix being decomposed.

3. The tissue regeneration construct (10, 20, 30) according claim 1 or claim 2, wherein at least either one of the cells comprised in the transplant body (11, 21, 31) and the cells comprised in the engraftment layer (15, 25, 35) are undifferentiated mesenchymal stem cells.

4. The tissue regeneration construct (10, 20, 30) according to claim 1 or claim 2, wherein at least either one of the cells comprised in the transplant body (11, 21, 31) and the cells comprised in the engraftment layer (15, 25, 35) are differentiated cells.

5. The tissue regeneration construct (10, 20, 30) according to claim 1 or claim 2, wherein at least either one of the cells comprised in the transplant body (11, 21, 31) and the cells comprised in the engraftment layer (15, 25, 35) are tissue precursor cells cultured in a state of being differentiated from stem cells.

6. The tissue regeneration construct (10, 20, 30) according to any one of claims 1 to 5, wherein the support (12) of the transplant body (11, 21, 31) is formed containing at least one selected from the group consisting of hydroxyapatite, apatite carbonate, β-TCP, OCP, and calcium phosphate.

7. The tissue regeneration construct (10, 20, 30) according to any one of claims 1 to 5, wherein the support (12) of the transplant body (11, 21, 31) is formed containing at least one selected from the group consisting of PLGA, PLLA, PLC, and artificial polymers having biocompatibility.

## Patentansprüche

1. Geweberegenerationskonstrukt (10, 20, 30), das ein auf eine Zielstelle zur Transplantation und Regeneration anzuwendendes Element ist, um Gewebe zu regenerieren, wobei das Geweberegenerationskonstrukt (10, 20, 30) umfasst:
einen Transplantatkörper (11, 21, 31); und
eine Einwachsschicht (15, 25, 35), die so angeordnet ist, dass sie zumindest einen Teil einer Außenfläche des Transplantatkörpers überlappt,
wobei:
der Transplantatkörper (11, 21, 31) einen Träger (12) und Zellen zum Regenerieren des Gewebes umfasst, wobei die Zellen in zumindest einem eines Raums zwischen den Trägern und eines durch eine Pore innerhalb des Trägers gebildeten Raums angeordnet sind,
die Einwachsschicht (15, 25, 35) Zellen zum Regenieren des Gewebes und ein Basismaterial umfasst, das Fibrin aus Blutplasma enthält, das durch eine Gerinnungsreaktion polymerisiert wurde, wobei das Basismaterial zur Aufnahme der Zellen vorgesehen ist,
das Basismaterial der Einwachsschicht gelatineartig ist, und
die Einwachsschicht (15, 25, 35) eine Schicht ist, in der kein Träger (12) vorhanden ist.

2. Geweberegenerationskonstrukt (10, 20, 30) nach Anspruch 1, wobei sich die in der Einpflanzschicht (15, 25, 35) umfassten Zellen in einem dispergierten Zustand befinden, wobei interzelluläre Matrix zersetzt ist.

3. Geweberegenerationskonstrukt (10, 20, 30) nach Anspruch 1 oder 2, wobei zumindest eines der Zellen, die im Transplantatkörper (11, 21, 31) umfasst sind, und der Zellen, die in der Einpflanzschicht (15, 25, 35) umfasst sind, undifferenzierte Mesenchymalstammzellen sind.

4. Geweberegenerationskonstrukt (10, 20, 30) nach Anspruch 1 oder 2, wobei zumindest eines der Zellen, die im Transplantatkörper (11, 21, 31) umfasst sind, und der Zellen, die in der Einpflanzschicht (15, 25, 35) umfasst sind, differenzierte Zellen sind.

5. Geweberegenerationskonstrukt (10, 20, 30) nach Anspruch 1 oder 2, wobei zumindest eines der Zellen, die im Transplantatkörper (11, 21, 31) umfasst sind, und der Zellen, die in der Einpflanzschicht (15, 25, 35) umfasst sind, Gewebevorläuferzellen sind, die in einem von Stammzellen differenzierten Zustand kultiviert wurden.

6. Geweberekonstruktionskonstrukt (10, 20, 30) nach einem der Ansprüche 1 bis 5, wobei der Träger (12) des Transplantatkörpers (11, 21, 31) gebildet wird, der zumindest eines enthält, das aus der Gruppe ausgewählt ist, bestehend aus Hydroxyapatit, Apatitcarbonat, β-TCP, OCT und Calciumphosphat.

7. Geweberekonstruktionskonstrukt (10, 20, 30) nach einem der Ansprüche 1 bis 5, wobei der Träger (12) des Transplantatkörpers (11, 21, 31) gebildet wird, der zumindest eines enthält, das aus der Gruppe ausgewählt ist, bestehend aus PLGA, PLLA, PLC und künstlichen Polymeren mit Biokompatibilität.

## Revendications

1. Construction pour régénération de tissu (10, 20, 30) qui est un élément à appliquer sur un site cible de transplantation et de régénération pour régénérer un tissu, la construction pour régénération de tissu (10, 20, 30) comprenant :
un corps de transplant (11, 21, 31) ; et
une couche de greffe (15, 25, 35) agencée de façon à chevaucher au moins une partie d'une surface externe du corps de transplant,
le corps de transplant (11, 21, 31) comprenant un support (12) et des cellules pour régénérer le tissu, les cellules étant agencées dans au moins l'un ou l'autre parmi un espace entre les supports et un espace formé par un pore à l'intérieur du support,
la couche de greffe (15, 25, 35) comprenant des cellules pour régénérer le tissu et un matériau de base contenant de la fibrine provenant de plasma sanguin polymérisé par réaction de coagulation, le matériau de base servant à retenir les cellules,
le matériau de base de la couche de greffe étant gélatineux, et
la couche de greffe (15, 25, 35) étant une couche dans laquelle le support (12) n'existe pas.

2. Construction pour régénération de tissu (10, 20, 30) selon la revendication 1, dans laquelle les cellules comprises dans la couche de greffe (15, 25, 35) sont dans un état dans lequel elles sont dispersées avec une matrice intercellulaire en cours de décomposition.

3. Construction pour régénération de tissu (10, 20, 30) selon la revendication 1 ou la revendication 2, dans laquelle au moins l'une ou l'autre parmi les cellules comprises dans le corps de transplant (11, 21, 31) et les cellules comprises dans la couche de greffe (15, 25, 35) sont des cellules souches mésenchymateuses non différenciées.

4. Construction pour régénération de tissu (10, 20, 30) selon la revendication 1 ou la revendication 2, dans laquelle au moins l'une ou l'autre parmi les cellules comprises dans le corps de transplant (11, 21, 31) et les cellules comprises dans la couche de greffe (15, 25, 35) sont des cellules différenciées.

5. Construction pour régénération de tissu (10, 20, 30) selon la revendication 1 ou la revendication 2, dans laquelle au moins l'une ou l'autre parmi les cellules comprises dans le corps de transplant (11, 21, 31) et les cellules comprises dans la couche de greffe (15, 25, 35) sont des cellules précurseurs de tissu cultivées dans un état dans lequel elles se différencient à partir de cellules souches.

6. Construction pour régénération de tissu (10, 20, 30) selon l'une quelconque des revendications 1 à 5, dans laquelle le support (12) du corps de transplant (11, 21, 31) est formé de façon à contenir au moins un choisi parmi le groupe constitué d'hydroxyapatite, de carbonate-apatite, de β-TCP, d'OCP et de phosphate de calcium.

7. Construction pour régénération de tissu (10, 20, 30) selon l'une quelconque des revendications 1 à 5, dans laquelle le support (12) du corps de transplant (11, 21, 31) est formé de façon à contenir au moins un choisi parmi le groupe constitué de PLGA, de PLLA, de PLC et de polymères artificiels ayant une biocompatibilité.
